Europäisches Patentamt

European Patent Office

Office européen des brevets

(11) Veröffentlichungsnummer: **0 335 056**
**A1**

(12) **EUROPÄISCHE PATENTANMELDUNG**

(21) Anmeldenummer: 88810218.3

(51) Int. Cl.⁴: **G02F 1/13 , A61F 9/06**

(22) Anmeldetag: 31.03.88

(43) Veröffentlichungstag der Anmeldung:
04.10.89 Patentblatt 89/40

(84) Benannte Vertragsstaaten:
AT BE CH DE ES FR GB GR IT LI LU NL SE

(71) Anmelder: OPTREL AG
Ebnater Strasse 84
CH-9630 Wattwil(CH)

(72) Erfinder: Bruhin, Rolf, c/o Bruhin Industrie
Elektronik
Kratzstrasse 35,
CH-8620 Wetzikon(CH)
Erfinder: Fürthbauer, Rupert,
Hauptgasse, 30
CH-9620 Lichtensteig(CH)

(74) Vertreter: Rottmann, Maximilian R.
c/o Rottmann, Maspoli + Zimmermann AG
Glattalstrasse 37
CH-8052 Zürich(CH)

(54) **Lichtfilter mit selbsttätiger Regelung der optischen Transmission.**

(57) Die Erfindung betrifft die Verbesserung eines Lichtfilters mit selbsttätiger Regelung der optischen Transmission. Es besitzt ein Filterelement (1) mit einer zwischen zwei transparenten Scheiben (2) und zugeordneten Polarisatoren angeordneten Flüssigkristallschicht (3), wobei das Lichtfilter durch Anlegen einer elektrischen Spannung variabler Grösse in der optischen Transmission veränderlich ist. Ein erster optischer Sensor (4) ist in Strahlungseinfallrichtung hinter dem Filterelement (1) angeordnet, und ein weiterer Sensor (8) ist vorgesehen, der neben oder in Strahlungseinfallrichtung vor dem Filterelement (1) angeordnet ist. Die beiden Sensoren sind an einen Regelstromkreis (16) angeschlossen, der eine SubtrahierSchaltung aufweist (15), welche das vom weiteren Sensor (8) gelieferte Signal vom Signal des optischen Sensors (4) oder das Signal des optischen Sensors (4) vom Signal des weiteren Sensors (8) subtrahiert, so dass das Ausgangssignal des Regelstromkreises (6) zumindest annähernd der Menge des sichtbaren Lichtes proportional ist. Für den weiteren Sensor (8) kann auch ein lediglich auf IR-Strahlung empfindlicher Sensor verwendet werden, oder wenn dieser auch für andere Strahlung empfindlich ist, kann diesem ein IR-Bandpassfilter vorgeschaltet werden.

FIG. 1

EP 0 335 056 A1

## LICHTFILTER MIT SELBSTTÄTIGER REGELUNG DER OPTISCHEN TRANSMISSION

Die vorliegende Erfindung bezieht sich auf ein Lichtfilter mit selbsttätiger Regelung der optischen Transmission nach dem Oberbegriff des Patentanspruches 1 bzw. des Patentanspruches 2.

Solche Lichtfilter werden z.B. als Schutzfilter in Schweisser-Schutzschilden, Schweisser-Schutzhelmen und dgl. verwendet, um die Bedienungsperson des Schweissgerätes vor der beim Schweissen auftretenden, äusserst hellen Strahlung zu schützen. Hingegen sind auch andere Anwendungen denkbar, z.B. bei Sonnenbrillen, Fensterscheiben für Gebäude, Auto- oder Flugzeugscheiben usw.

Eine Sichtscheibe mit automatischer Regelung der Lichtdurchlässigkeit ist unter anderem aus der DE-OS 24 42 998 bekannt. Dort wird eine sogenannte LC-Zelle, also ein Flüssigkristall-Element zur Lichtabschirmung verwendet. Die hinter der Sichtscheibe ankommende Lichtmenge wird von einem Photosensor gemessen und als Regelgrösse einer Schaltung zugeführt, welche die Ansteuerspannung für die Flüssigkristallschicht derart einstellt, dass die durchgelassene Lichtmenge einem vorgewählten Sollwert entspricht.

Theoretisch mag eine solche Anordnung funktionieren, doch hat es sich herausgestellt, dass das Auftreten von IR-Strahlung diesen Regelmechanismus empfindlich stören kann. Da aber gerade bei Schweisser-Anwendungen vermehrt und nahezu immerzu eine starke IR-Strahlung auftritt, eignet sich die in jener DE-OS vorgeschlagene Vorrichtung nur bedingt für Schweisser-Schutzschilder oder -Helme, da starke Sonneneinstrahlung, insbesondere aber die Arbeit eines benachbarten Schweisser-Kollegen die Regelung unerwünschterweise beeinträchtigen kann, weil dabei eine starke IR-Strahlung auftritt, die das Schutzschild bzw. den Helm unbeabsichtigt dunkelsteuern wird.

Die ebenfalls vorhandene, unsichtbare UV-Strahlung stört bei einer Anordnung mit hinter der LC-Zelle plaziertem Sensor weniger oder gar nicht, da LC-Zellen bekanntlich IR-Strahlung nahezu ungehindert durchlassen, UV-Strahlung aber weitgehend abschirmen, besonders wenn sie wie üblich mit vor- und nachgeschalteten Polarisatoren versehen sind. Dazu kommt, dass gebräuchliche Photosensoren, neben ihrer Empfindlichkeit im sichtbaren Spektralbereich, für UV-Strahlung wenig oder gar nicht, für IR-Strahlung hingegen ausgesprochen empfindlich sind.

Es ist die Aufgabe der vorliegenden Erfindung, ein Lichtfilter der gattungsgemässen Art so zu verbessern, dass es in Bezug auf sein Regelverhalten weitgehend nur noch auf sichtbares Licht, nicht oder kaum aber auf IR-Strahlung anspricht, welch letztere für den Menschen bdeutend weniger schädlich ist.

Nach einem ersten Erfindungsgedanken wird diese Aufgabe bei einem Lichtfilter der im Oberbegriff des Patentanspruchs 1 definierten Art, d.h. bei einer Anordnung mit einem einzelnen bezüglich seiner optischen Transmission des sichtbaren Lichtes regelbaren Lichtfilter-Element, dadurch gelöst, dass ein weiterer Sensor vorgesehen ist, der neben oder in Strahlungseinfallrichtung vor dem Filterelement angeordnet ist, und dass der Regelstromkreis eine Subtrahier-Schaltung aufweist, welche das vom weiteren Sensor gelieferte Signal vom Signal des optischen Sensors oder das Signal des optischen Sensors vom Signal des weiteren Sensors subtrahiert, so dass das Ausgangssignal des Regelstromkreises zumindest annähernd der Menge des vor dem Filterelement vorhandenen, sichtbaren Lichtes proportional ist.

Bei einer Anordnung mit zwei hintereinandergeschalteten, bezüglich ihrer optischen Transmission des sichtbaren Lichtes regelbaren Lichtfilter-Elementen wird die erfindungsgemässe Aufgabe gemäss Patentanspruch 2 dadurch gelöst, dass der optische Sensor in Strahlungseinfallrichtung funktionell hinter dem ersten Filterelement angeordnet ist, dass ein weiterer Sensor vorgesehen ist, der neben oder in Strahlungseinfallrichtung vor dem ersten Filterelement angeordnet ist, und dass der Regelstromkreis eine Subtrahier-Schaltung aufweist, welche das vom weiteren Sensor gelieferte Signal vom Signal des optischen Sensors oder das Signal des optischen Sensors vom Signal des weiteren Sensors subtrahiert, so dass das Ausgangssignal des Regelstromkreises zumindest annähernd der Menge des sichtbaren Lichtes proportional ist.

Aus der DE-OS 33 40 877 ist zwar eine Schaltungsanordnung zur fremdlichabhängigen Regelung von Lichtschranken bekannt geworden, bei der ein zweiter Sensor zur Erfassung des Umgebungslichtes vorhanden ist. Je nach Stärke desselben wird die Strahlstärke der Lichtschranke nachgeregelt. Eine Ausschaltung von Einflüssen eventuell vorhandener IR-Strahlung ist nicht beabsichtigt und auch gar nicht möglich, da bei jener Schaltungsanordnung beide lichtempfindlichen Elemente immer dem gleichen Umgebungslicht ausgesetzt sind.

Im Gegensatz dazu ist bei der erfindungsgemässen Lösung sichergestellt, dass durch die Subtraktion der Einfluss der IR-Strahlung ausgeschaltet ist, so dass sich die Regelschaltung nur nach der Menge des sichtbaren Lichtes richtet. Wie schon erwähnt, hat die UV-Strahlung nur einen geringen Einfluss und kann gegebenenfalls noch durch Vorschaltung eines UV-Sperrfilters vor den einen oder vor beide Sensoren weiter verringert werden.

Im Interesse der konstruktiven Einfachheit kann es vorteilhaft sein, wenn der genannte optische Sensor und der weitere Sensor gleich sind. Dies hat zudem auch den Vorteil, dass deren Empfindlichkeit gleich ist, was den Aufwand in der Regelschaltung verringert und das Regelverhalten verbessert. Bei dieser Lösung wird ein, je nach Schaltungsart, negatives oder positives Steu ersignal erhalten, welches der Differenz zwischen tatsächlich vorhandenem Licht und dem gefiltertem, sichtbaren Licht entspricht, somit der Menge des hinter dem Lichtfilter-Element vorhandenen, sichtbaren Lichtes ohne IR-Anteil proportional ist.

Eine andere Lösung kann darin bestehen, dass der hinter dem Lichtfilter-Element angeordnete Sensor für IR-Strahlung und sichtbares Licht, der weitere Sensor aber nur für IR-Strahlung empfindlich ist. Nach der Subtraktion wird je nach Schaltungsauslegung ein positives oder negatives Signal erhalten, welches direkt proportional zum Anteil des sichtbaren Lichtes, ohne IR-Strahlung, ist. Der Nachteil hierbei ist, dass zwei verschiedene, möglicherweise unterschiedlich empfindliche Sensoren oder Sensoren mit unterschiedlicher Kennlinie verwendet werden müssen; dies kann aber dadurch kompensiert werden, dass dem einen oder anderen oder beiden Sensoren ein regelbarer Verstärker nachgeschaltet wird. Jedenfalls aber geniesst man den Vorteil, dass die anschliessende Regelschaltung einfacher wird.

Weitere Ausführungsmöglichkeiten und Weiterbildungen des Erfindungsgegenstandes sind in den abhängigen Ansprüchen definiert.

Im folgenden wird die Erfindung näher erläutert, unter Bezugnahme auf die beiliegenden Zeichnungen, in welchen schematisch einige Ausführungsbeispiele dargestellt sind. Im einzelnen zeigen:

Fig. 1 ein erstes Ausführungsbeispiel mit einem einzelnen Filterelement;

Fig. 2 eine abgewandelte Ausführungsform des ersten Ausführungsbeispieles;

Fig. 3 ein zweites Ausführungsbeispiel mit zwei hintereinandergeschalteten Filterelementen; und

Fig. 4 eine abgewandelte Ausführungsform des zweiten Ausführungsbeispieles.

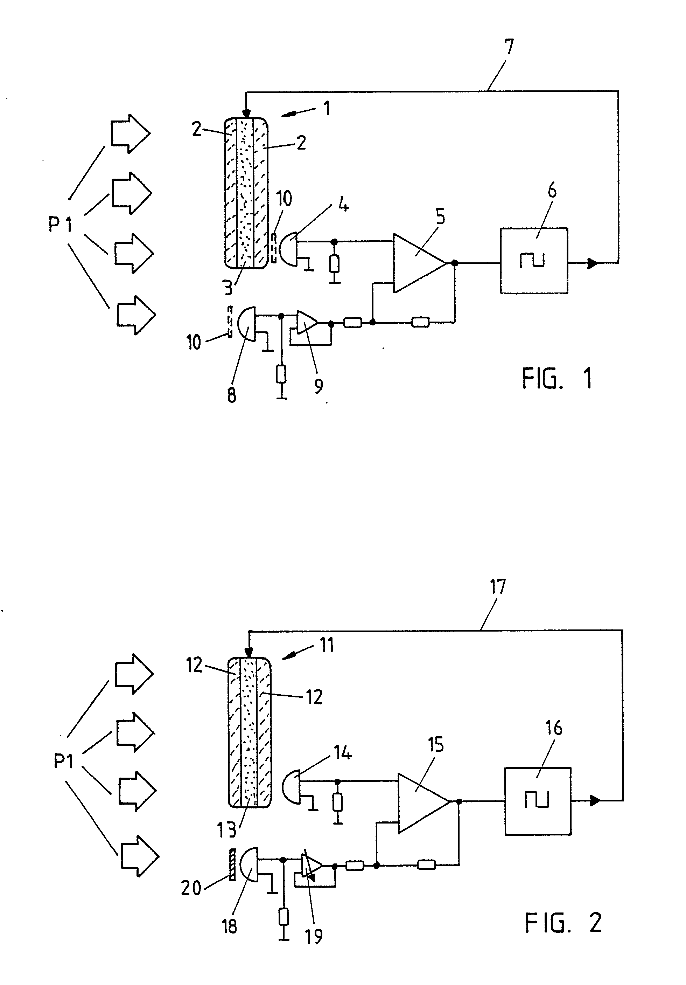

Gemäss Fig. 1 umfasst das Lichtfilter gemäss der Erfindung beispielsweise eine LC-Zelle, gesamthaft mit 1 bezeichnet, d.h. ein Flüssigkristall-Element, das im Prinzip durch zwei parallele Glasscheiben 2 mit einer dazwischen liegenden Flüssigkristall-Schicht 3 gebildet ist. Die Glasscheiben können selbst als Polarisator wirken oder es können den LC-Elementen separate Polarisatoren zugeordnet sein. Solche Elemente sind bekannt und zeichnen sich dadurch aus, dass ein solches Filterelement bei Anlegen einer variablen Wechselspannung an die Flüssigkristall-Schicht 3 von einem weitgehend transparenten Zustand in einen weitgehend lichtundurchlässigen Zustand umgesteuert werden kann.

Hinter der LC-Zelle 1 ist ein Photosensor 4 angeordnet, der die von der Zelle 1 durchgelassene Strahlung auffängt und ein Ausgangssignal liefert, das der empfangenen Strahlungsintensität proportional ist. Ueber einen Differentialverstärker 5, auf den nachstehend noch eingegangen werden wird, gelangt das Ausgangssignal des Sensors 4 zu einem Regelstromkreis 6, welcher das Signal aufbereitet und seinerseits ein Ausgangs-Steuersignal über eine Leitung 7 an die LC-Zelle 1 liefert. Die Anordnung ist dabei so getroffen, dass die Zelle 1 dann dunkelgesteuert wird, wenn der Sensor 4 viel Licht empfängt, und umgekehrt; eine solche Vorrichtung ist bekannt und braucht hier nicht näher erläutert zu werden.

Die in Fig. 1 gezeigte Anordnung umfasst ferner einen weiteren Sensor 8, der im gezeigten Fall neben der Zelle 1 angeordnet ist; genausogut könnte er vor der Zelle 1 angeordnet sein, da lediglich wichtig ist, dass er stets die Gesamtmenge der vorhandenen Strahlung, ohne Beeinflussung durch die Zelle 1, empfangen kann. Der Sensor 8 liefert ein von der empfangenen Strahlung abhängiges Ausgangssignal an einen Impedanzwandler 9, dessen Ausgang an den zweiten Eingang des Differentialverstärkers 5 angeschlossen ist. Gegebenenfalls können dem Sensor 4 und/oder dem Sensor 8 UV-Sperrfilter vorgeschaltet sein, um eventuell störende UV-Strahlung abzuhalten.

Die Funktion dieser Schaltung ist wie folgt:

Strahlung, die z.B. von einer Schweiss-Stelle erzeugt wird, also eine (verhältnismässig starke) IR-Komponente, eine Komponente sichtbaren Lichtes und eventuell auch eine UV-Komponente enthält, fällt in Richtung der Pfeile P1 auf die Vorrichtung. Die Zelle 1 ist in offenem Zustand und der Sensor 4 nimmt nahezu die Gesamtheit der vorhandenen Strahlung auf. Allerding ist festzuhalten, dass eine LC-Zelle, selbst im offenen Zustand, bereits wie ein UV-Sperrfilter wirkt, so dass in der Praxis zunächst der gesamte Anteil von IR-Strahlung und sichtbarem Licht auf den Sensor 4 fällt. Der Regelstromkreis 6 ist in bekannter Weise so ausgelegt, dass er sofort ein Ausgangssignal an die Zelle 1 liefert, welches innerhalb von ms bewirkt, dass die Zelle 1 dunkelsteuert, d.h. ihre Strahlungsdurchlässigkeit vermindert. Eine gegebenenfalls vorgesehene Steuerschaltung erlaubt dabei, den Regelstromkreis 6 auf einen bestimmten, vorwählbaren Abdunkelungswert einzustellen.

Die Eigenart einer solchen Anordnung besteht aber darin, dass auch bei alleinigem Auftreten von IR-Strahlung bzw. bei einem starken Anteil einer solchen Wellenlänge ein Ansprechen des Regelkreises und damit eine Abdunkelung für sichtbares Licht erfolgt; dies ist aber in vielen Fällen höchst unerwünscht, Z.B.

3

bei starker Sonneneinstrahlung oder bei einer IR-Strahlung, die durch einen benachbarten Arbeitsplatz verursacht wird.

Hier Abhilfe zu schaffen ist die Aufgabe der vorliegenden Erfindung. Dies geschieht beim hier zunächst diskutierten, in Fig. 1 schematisch dargestellten Ausführungsbeispiel auf folgende Weise:

Es wird davon ausgegangen, dass beide Sensoren, nämlich der Sensor 4 und der Sensor 8, identisch sind, d.h. für IR-Strahlung und für sichtbares Licht gleich empfindlich sind. Der Sensor 4 empfängt die gesamte oder nahezu die gesamte IR-Strahlung und einen Anteil des sichtbaren Lichtes, wenn die LC-Zelle dunkelgesteuert ist. Der Sensor 8 hingegen empfängt die gesamte IR-Strahlung sowie die gesamte Strahlung im sichtbaren Spektralbereich, da er gemäss der Erfindung vor oder neben der LC-Zelle 1 angeordnet ist. Die UV-Strahlungsanteile können dabei praktisch vernachlässigt werden. Es sei:

| IR | = | Anteil der IR-Strahlung |
| $S_v$ | = | Anteil der sichtbaren Strahlung nach der LC-Zelle |
| $S_k$ | = | Anteil der sichtbaren Strahlung vor der LC-Zelle. |
| $A_{o1}$ | = | Proportionalitätsfaktor des Ausgangssignals nach der Subtrahierschaltung. |

Dann gilt, je nachdem, ob der Sensor 4 an den nicht-invertierenden Eingang und der Sensor 8 an den invertierenden Eingang des Differentialverstärkers 5 angeschlossen ist, oder umgekehrt:

$$(S_v + IR) - (S_k + IR) = S_v - S_k = -A_{o1}, \text{ oder}$$
$$-(S_v + IR) + (S_k + IR) = -S_v + S_k = +A_{o1}.$$

In beiden Fällen wird also ein vom IR-Strahlungsanteil unabhängiger Faktor erhalten, der zur Steuerung der LC-Zelle 1 her angezogen wird und der zudem vom Anteil der sichtbaren Strahlung nach der Zelle 1 beeinflusst ist, somit als Regelgrösse herangezogen werden kann.

Die vorstehend diskutierte Anordnung hat den Vorteil, dass zwei identische Sensoren (4 und 8) verwendet werden können, deren Kennlinien auch gleich oder zumindest sehr ähnlich sind. Allerdings ist der Aufwand zur Auswertung des Ausgangssignales $A_{o1}$ bzw. $-A_{o1}$ etwas grösser.

Um diesen Aufwand zu vermeiden besteht die Möglichkeit, für die Sensoren 4 und 8 zwei unterschiedliche Elemente zu verwenden: Der Sensor 4 hinter der Zelle 1 ist für IR-Strahlung und für sichtbares Licht empfindlich; der Sensor 8 neben oder vor der Zelle 1 hingegen nur für IR-Strahlung. Dann gilt, unter Zugrundelegung der vorstehenden Definition, widerum in Abhängigkeit davon, wie die beiden Sensoren an den Differentialverstärker angeschlossen sind:

$$(S_v + IR) - (IR) = S_v = A_{o2}, \text{ oder}$$
$$-(S_v + IR) + (IR) = -S_v = -A_{o2}.$$

Hier sind die Ausgangssignale $A_{o2}$ bzw. $-A_{o2}$ ebenfalls frei von jedem IR-Anteil und zudem direkt der Menge des hinter die Zelle 1 gelangenden, sichtbaren Lichtes proportional, können also einfach und direkt in der Regelschaltung verarbeitet werden.

Bei dieser Anordnung können zwei verschiedene Sensoren als Sensoren 4 und 8 verwendet werden, oder es können, gemäss Fig. 2, identische Sensoren 14 und 18 Anwendung finden, wobei dem Sensor 18 ein IR-Bandpassfilter 20 vorgeschaltet werden. Die restliche Anordnung mit LC-Zelle 11, bestehend aus zwei parallelen Glasplatten 12, einer dazwischenliegenden Flüssigkristallschicht 13, gegebenenfalls (nicht dargestellten) separaten Polarisatoren, dem dahinter angeordneten Photosensor 14, dem Differentialverstärker 15, dem Regelstromkreis 16, der über eine Leitung 17 mit der Zelle 11 verbunden ist, und dem Impedanzwandler/Verstärker 19 ist im Prinzip genau gleich. Allerdings kann es sich hier empfehlen, den Schaltkreis 19 bezüglich der Verstärkung regelbar auszubilden, um allfällige Empfindlichkeitsverluste, hervorgerufen durch das Filter 20, ausgleichen zu können.

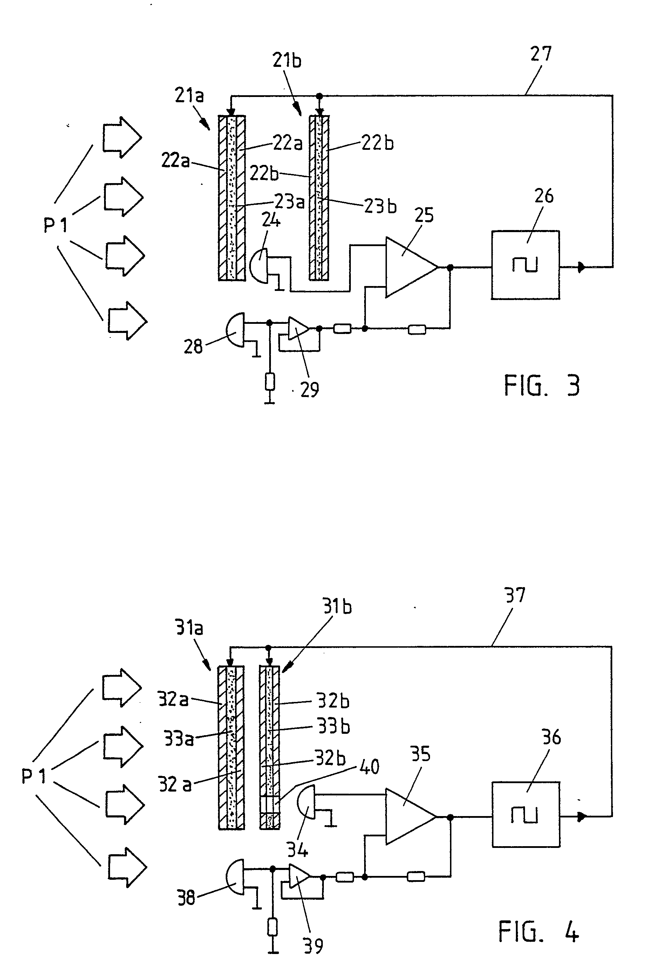

In der Fig. 3 ist eine zweite Ausführungsform dargestellt. Im Gegensatz zur Ausführung gemäss Fig.1 und 2 sind hier zwei hintereinandergeschaltete LC-Zellen 21a und 21b vorgesehen, um eine manchmal wünschenswerte, stärkere Lichtdämpfung zu erreichen. Im übrigen sind diese sonst gleich aufgebaut, mit einer Flüssigkristallschicht 23a bzw. 23b zwischen jeweils zwei parallelen Glasscheiben 22a bzw. 22b, die selbst als Polarisatoren wirken, bze. mit (nicht dargestellten, getrennten, vorund nachgeschalteten Polarisatoren. Der Photosensor 24 ist in Strahlungseinfallrichtung P1 hinter der ersten Zelle 21a, jedoch vor der Zelle 21b angeordnet, damit eine genügende Rest lichtmenge auf den Sensor fällt, die zur einwandfreien Ansteuerung des Regelstromkreises 26 erforderlich ist.

In entsprechender Weise ist ein weiterer Sensor 28 neben oder vor der ersten Zelle 21a angeordnet, die, wie schon erwähnt, entweder das ganze Strahlungsspektrum oder nur den IR-Anteil aufnimmt und ihr Ausgangssignal über einen gegebenenfalls regelbaren Impedanzwandler/Verstärker 29 an einen Differentialverstärker 25 leitet. Der Ausgang des Regelstromkreises 26 ist über eine Leitung 27 an die beiden

4

hintereinandergeschalteten Zellen 21a und 21b geführt, wobei gegebenenfalls (nicht gezeigte) Mittel zur gegenphasigen Ansteuerung der Zellen vorgesehen sein können.

In Bezug auf die Spektral-Empfindlichkeit der Zellen 24 und 28 bzw. allfällig vorgeschalteter Filter (nicht dargestellt) gilt sinngemäss das vorher Gesagte.

Wenn es aus physikalischen Gründen oder aus kommerziellen Überlegungen unmöglich ist, den Sensor 24 zwischen den Zellen 21a und 21b anzuordnen, kommt eine Lösung gemäss Fig. 4 in Betracht. Hier sind, in Strahlungseinfallrichtung P1 gesehen, zwei hintereinandergeschaltete, eng nebeneinander angeordnete LC-Zellen 31a und 31b vorgesehen, die jeweils wiederum durch zwei parallele Glasplatten 32a bzw. 32b sowie eine dazwischenliegende Flüssigkristallschicht 33a bzw. 33b gebildet sind. Bezüglich der Polarisatoren gilt sinngemäss das vorher Gesagte.

Damit der Photosensor 34 wirkungsmässig hinter der ersten Zelle 31a, physisch aber hinter der zweiten Zelle 31b angeordnet werden kann, ist die zweite Zelle 31b mit einer Oeffnung 40 versehen, die stets offen bleibt, also auch bei Dunkelsteuerung der Zelle 31b kein verändertes optisches Transmissionsverhalten zeigt. Dies ist immer dann vorteilhaft, wenn die beiden LC-Zellen 31a und 31b, im Gegensatz zur schematischen Darstellung in der Zeichnung, sehr eng oder dicht aneinanderliegend angeordnet sind. Die kleine Oeffnung 40 am Rand der Zelle 31b stört dabei das Verhalten und die Wirkung der Gesamtanordnung kaum oder überhaupt nicht. Im übrigen ist der Aufbau der Anordnung mit weiterem Sensor 38, Impedanzwandler/Verstärker 39, Differentialverstärker 35 und über Leitung 37 an die Zellen 31a und 31b angeschlossenem Regelstromkreis 36 gleich wie schon beschrieben.

Selbstverständlich kann auch bei der Anordnung gemäss Fig.3 bzw. gemäss Fig.4 für die Sensoren 24 bzw. 34 einerseits und 28 bzw. 38 andererseits der gleiche Typ, gegebenenfalls mit (nicht dargestelltem) IR-Bandpassfilter vor dem Sensor 28 bzw. 38, oder aber ein unterschiedlicher Typ verwendet werden, d.h. als Sensor 24 bzw. 34 eine für IR und für sichtbares Licht empfindliche Ausführung, als Sensor 28 bzw. 38 hingegen eine lediglich IR-empfindliche Ausführung. Auch das bezüglich einstellbarer Verstärkung des Impedanzwandlers/Verstärkers 25 bzw. 35 vorher Gesagte gilt sinngemäss.

Schliesslich soll nicht unerwähnt bleiben, dass den LC-Zellen Polarisationsfilter vorgeschaltet und nachgeschaltet werden müssen, z.B. in der Art, wie dies durch den Stand der Technik bekannt geworden ist, beispielsweise durch die US-PS Nr. 4,039,254.

Wesentlich ist im erfindungsgemässen Zusammenhang, dass der Einfluss der IR-Strahlung auf die Regelschaltung ferngehalten wird, dass gleichsam ein IR-Sperrfilter elektrisch simuliert wird. Damit ist eine wirkungsvolle, zuverlässige Regelung der Lichtdurchlässigkeit des Lichtfilters, bestehe es nun aus einer oder aus zwei hintereinandergeschalteten Zellen, stets gewährleistet, auch wenn in der Umgebung starke IR-Strahlung herrscht, da der Regelkreis nurmehr noch auf das sichtbare Licht anspricht; die ausserdem vorhandene UV-Strahlung stört kaum, da die meisten Photosensoren, wie schon erwähnt, darauf kaum ansprechen und da eine Flüssigkristallzelle an sich schon ein gutes UV-Sperrfilter darstellt.

## Ansprüche

1. Lichtfilter mit selbsttätiger Regelung der optischen Transmission, welches ein bezüglich seiner optischen Transmission des sichtbaren Lichtes regelbares Filterelement aufweist, und mit einem optischen Sensor, welcher das einfallende und/oder das Umgebungs-Licht erfasst und an einen Regelstromkreis angeschlossen ist, welcher das zur Regelung der optischen Transmission des Filterelementes benötigte, elektrische Signal in Abhängigkeit der auf den Sensor fallenden Strahlung erzeugt und der in Strahlungseinfallrichtung hinter dem Filterelement angeordnet ist, dadurch gekennzeichnet, dass ein weiterer Sensor 8; 18) vorgesehen ist, der neben oder in Strahlungseinfallrichtung vor dem Filterelement (1; 11) angeordnet ist, und dass der Regelstromkreis (6; 16)) eine Subtrahier-Schaltung (5; 15) aufweist, welche das vom weiteren Sensor (8; 18) gelieferte Signal vom Signal des optischen Sensors (4; 14) oder das Signal des optischen Sensors (4; 14) vom Signal des weiteren Sensors (8; 18) subtrahiert, so dass das Ausgangssignal des Regelstromkreises (6; 16) zumindest annähernd der Menge des vor dem Filterelement vorhandenen, sichtbaren Lichtes proportional ist.

2. Lichtfilter mit selbsttätiger Regelung der optischen Transmission, welches zwei bezüglich ihrer optischen Transmission des sichtbaren Lichtes regelbare Filterelemente aufweist, und mit einem optischen Sensor, welcher das einfallende und/oder das Umgebungs-Licht erfasst und an einen Regelstromkreis angeschlossen ist, welcher das zur Regelung der optischen Transmission der Filterelemente benötigte, elektrische Signal in Abhängigkeit der auf den Sensor fallenden Strahlung erzeugt, dadurch gekennzeichnet, dass der optische Sensor (24; 34) in Strahlungseinfallrichtung funktionell hinter dem ersten Filterelement (21a; 31a) angeordnet ist, dass ein weiterer Sensor (28; 38) vorgesehen ist, der neben oder in Strahlungseinfallrichtung vor dem ersten Filterelement (21a; 31a) angeordnet ist, und dass der Regelstromkreis (26; 36) eine Subtrahier-Schaltung (25; 35) aufweist, welche das vom weiteren Sensor (28; 38) gelieferte Signal vom Signal des optischen Sensors (24; 34) oder das Signal des optischen Sensors (24; 34) vom Signal des weiteren Sensors (28; 38) subtrahiert, so dass das Ausgangssignal des Regelstromkreises (26; 36) zumindest annähernd der Menge des vor dem Filterelement vorhandenen, sichtbaren Lichtes proportional ist.

3. Lichtfilter nach Anspruch 1 oder 2, dadurch gekennzeichnet, dass der genannte optische Sensor (4; 14; 24; 34) und der weitere Sensor (8; 18; 28; 38) gleich sind.

4. Lichtfilter nach Anspruch 3, dadurch gekennzeichnet, dass der genannte optische Sensor (4; 14; 24; 34) und der weitere Sensor (8; 18; 28; 38) für IR-Strahlung und für sichtbares Licht empfindlich sind.

5. Lichtfilter nach Anspruch 1 oder 2, dadurch gekennzeichnet, dass der genannte optische Sensor (4; 14; 24; 34) für IR-Strahlung und für sichtbares Licht empfindlich ist, der weitere Sensor (8; 18; 28; 38) hingegen nur für IR-Strahlung.

6. Lichtfilter nach Anspruch 1 oder 2, dadurch gekennzeichnet, dass der genannte optische Sensor (4; 14; 24; 34) und der weitere Sensor (8; 18; 28; 38) gleich sind, und dass dem weiteren Sensor (8; 18; 28; 38) ein optisches IR-Bandpassfilter (20) vorgeschaltet ist.

7. Lichtfilter nach einem der Ansprüche 3 bis 6, dadurch gekennzeichnet, dass der genannte optische Sensor (4; 14; 24; 34) und/oder der weitere Sensor (8; 18; 28; 38) über einen in der Verstärkung regelbaren Verstärker bzw. Impedanzwandler (9; 19; 29; 39) an die Subtrahierschaltung (5; 15; 25; 35) des Regelstromkreises (6; 16; 26; 36) angeschlossen ist bzw. sind.

8. Lichtfilter nach einem der Ansprüche 3 bis 6, dadurch gekennzeichnet, dass dem genannten optischen Sensor (4; 14; 24; 34) und/oder dem weiteren Sensor (8; 18; 28; 38) ein Neutraldichtefilter für sichtbares Licht vorgeschaltet ist bzw. sind.

9. Lichtfilter nach einem der vorangehenden Ansprüche, dadurch gekennzeichnet, dass dem genannten optischen Sensor (4; 14; 24; 34) und/oder dem weiteren Sensor (8; 18; 28; 38) ein UV-Sperrfilter (10) vorgeschaltet ist bzw. sind.

10. Lichtfilter nach Anspruch 2, dadurch gekennzeichnet, dass das zweite Filterelement (31b) einen bezüglich der optischen Transmission unveränderlichen Teilbereich (40) aufweist und dass der optische Sensor (34) in Strahlungseinfallrichtung hinter dem zweiten Filterelement (31b) und hinter diesem unveränderlichen Teilbereich (40) angeordnet ist.

11. Lichtfilter nach einem der Ansprüche 3 oder 4, dadurch gekennzeichnet, dass die Subtrahierschaltung (5; 15; 25; 35) einen Differenzverstärker enthält, an dessen nicht-invertierenden Eingang der genannte optische Sensor (4; 14; 24; 34) und an dessen invertierenden Eingang der weitere Sensor (8; 18; 28; 38) angeschlossen sind, so dass dem Regelstromkreis (6; 16; 26; 36) ein negatives, dem sichtbaren Strahlungsanteil proportionales, von der IR-Strahlung unabhängiges Signal zugeführt ist.

12. Lichtfilter nach einem der Ansprüche 3 oder 4, dadurch gekennzeichnet, dass die Subtrahierschaltung (5; 15; 25; 35) einen Differenzverstärker enthält, an dessen invertierenden Eingang der genannte optische Sensor (4; 14; 24; 34) und an dessen nicht-invertierenden Eingang der weitere Sensor (8; 18; 28; 38) angeschlossen sind, so dass dem Regelstromkreis (6; 16; 26; 36) ein positives, dem sichtbaren Strahlungsanteil proportionales, von der IR-Strahlung unabhängiges Signal zugeführt ist.

13. Lichtfilter nach Anspruch 5, dadurch gekennzeichnet, dass die Subtrahierschaltung (5; 15; 25; 35) einen Differenzverstärker enthält, an dessen nicht-invertierenden Eingang der genannte optische Sensor (4; 14; 24; 34) und an dessen invertierenden Eingang der weitere Sensor (8; 18; 28; 38) angeschlossen sind, so dass dem Regelstromkreis (6; 16; 26; 36) ein negatives, dem vom Filterelement (1; 11; 21a; 31a) durchgelassenen, sichtbaren Strahlungsanteil direkt proportionales, von der IR-Strahlung unabhängiges Signal zugeführt ist.

14. Lichtfilter nach Anspruch 5, dadurch gekennzeichnet, dass die Subtrahierschaltung (5; 15; 25; 35) einen Differenzverstärker enthält, an dessen invertierenden Eingang der genannte optische Sensor (4; 14; 24; 34) und an dessen invertierenden Eingang der weitere Sensor (8; 18; 28; 38) angeschlossen sind, so dass dem Regelstromkreis (6; 16; 26; 36) ein positives, dem vom Filterelement (1; 11; 21a; 31a) durchgelassenen, sichtbaren Strahlungsanteil direkt proportionales, von der IR-Strahlung unabhängiges Signal zugeführt ist.

FIG. 1

FIG. 2

FIG. 3

FIG. 4

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | EP-A-0 091 514 (EGGENSCHWILER) * Figuren 1-5; Seite 3, Zeile 1 - Seite 4, Zeile 23 * --- | 1,2,6,9 ,10 | G 02 F 1/13 A 61 F 9/06 |
| A | US-A-4 491 390 (TONG-SHEN) * Figuren 1-3; Spalte 1, letzter Absatz - Spalte 2, Zeile 35 * --- | 1,2 | |
| A | FR-A-2 530 039 (CUVELIER et al.) * Patentansprüche 1,4,5; Figur 9 * --- | 1 | |
| E | DE-C-3 634 508 (FÜRTHBAUER) * Das ganze Dokument * ----- | 1-14 | |

RECHERCHIERTE
SACHGEBIETE (Int. Cl.4)

G 02 F
A 61 F

Der vorliegende Recherchenbericht wurde für alle Patentansprüche erstellt

| Recherchenort | Abschlußdatum der Recherche | Prüfer |
|---|---|---|
| DEN HAAG | 29-11-1988 | CONRAD V.HEYDENDORFF K. |

KATEGORIE DER GENANNTEN DOKUMENTE

X : von besonderer Bedeutung allein betrachtet
Y : von besonderer Bedeutung in Verbindung mit einer
    anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
    nach dem Anmeldedatum veröffentlicht worden ist
D : in der Anmeldung angeführtes Dokument
L : aus andern Gründen angeführtes Dokument
............................................................
& : Mitglied der gleichen Patentfamilie, übereinstimmendes
    Dokument

EPO FORM 1503 03.82 (P0403)